# EUROPÄISCHE PATENTANMELDUNG

(11) **EP 2 051 096 A2**
(43) Veröffentlichungstag der Anmeldung: **22.04.2009**
(21) Anmeldenummer: 08162248.2
(22) Anmeldetag: 12.08.2008
(51) Int. Cl.: G01S 5/16, G01S 15/89, A61B 5/103, A61B 19/00

(54) **Verfahren und Vorrichtung zur Bestimmung der Frontalebene des Beckenknochens**

(30) Priorität: 17.10.2007 DE 102007049671
(71) Anmelder: Aesculap AG, 78532 Tuttlingen (DE); Universität Ulm, 89081 Ulm (DE)
(72) Erfinder: Kozak, Josef, 78532, Tuttlingen (DE); Keppler, Peter, 89073, Ulm (DE)
(74) Vertreter: Hoeger, Stellrecht & Partner Patentanwälte

(57) **Zusammenfassung**

Um bei einem Verfahren zur Bestimmung der Beckeneingangsebene des Beckenknochens, die definiert wird durch folgende drei Punkte des Beckenknochens:
Punkt A :
spina iliaca anterior superior links
Punkt B :
spina iliaca anterior superior rechts
Punkt C :
symphysis pubis
durch nichtinvasive Bestimmung der Lage einer der beiden Punkte A oder B und des Punktes C diese Bestimmung auch durchführen zu können, wenn der Punkt A oder Punkt B nicht zugänglich sind, wird vorgeschlagen, dass man zusätzlich nichtinvasiv die Lage der folgenden Punkte des Beckenknochens bestimmt:
Punkt D :
spina iliaca posterior superior links
Punkt E :
spina iliaca posterior superior rechts
und dass man aus der Lage der nichtinvasiv bestimmten Punkte A oder B, C, D, E die Lage der Beckeneingangsebene berechnet.

## Beschreibung

Die Erfindung betrifft ein Verfahren zur Bestimmung der Beckeneingangsebene des Beckenknochens, die definiert wird durch folgende drei Punkte des Beckenknochens:
- Punkt A :: spinal iliac anterior superior links
- Punkt B :: spinal iliac anterior superior recites
- Punkt C :: symphysis pubis
durch nichtinvasive Bestimmung der Lage einer der beiden Punkte A oder B und des Punktes C. Außerdem betrifft die Erfindung eine Vorrichtung zur Durchführung dieses Verfahrens.

Beim Einsetzen von Implantaten und bei anderen Operationen ist es häufig notwendig, die anatomischen Gegebenheiten in abstrakter Weise zu beschreiben, um dadurch die anatomischen Gegebenheiten in mathematischen Modellen erfassen zu können, beispielsweise in Verbindung mit bekannten Navigationssystemen und mit der Bearbeitung der dadurch gewonnenen Daten in Datenverarbeitungsanlagen.

So ist es beispielsweise bekannt (DE 10 2005 003317 A1), den Beckenknochen dadurch zu beschreiben, dass eine Beckeneingangsebene definiert wird, die durch drei markante Punkte des Beckenknochens definiert ist, nämlich die folgenden Punkte:
- Punkt A :: linke spina iliaca anterior superior (linker vorderer oberer Darmbeinstachel)
- Punkt B :: rechte spina iliaca anterior superior (rechter vorderer oberer Darmbeinstachel)
- Punkt C :: symphysis pubis (Schambein).

Diese markanten Punkte können beispielsweise perkutan ertastet werden, es ist auch möglich, diese Punkte in anderer Weise nichtinvasiv festzustellen, beispielsweise durch einen Ultraschall-Sensor, durch Röntgenaufnahmen oder durch andere bildgebende Verfahren, beispielsweise computertomographische Verfahren.

In allen Fällen können die markanten Punkte verwendet werden, um daraus die Beckeneingangsebene zu beschreiben, so dass diese Beckeneingangsebene für die Berechnung der Lage des Beckenknochens in nachfolgenden Berechnungen verwendet werden kann.

Voraussetzung für dieses Verfahren ist allerdings, dass die drei markanten Punkte auch erreichbar sind, beispielsweise beim perkutanen Ertasten oder bei Anwendung eines vorzugsweise navigierten Ultraschallsensors. Dies ist aber nicht in allen Fällen möglich, beispielsweise ist bei einer Seitenlage eines Patienten auf einem Operationstisch immer nur einer der Punkte A oder B erreichbar, also nur entweder die linke oder die rechte spina iliaca anterior superior. Damit scheidet eine Bestimmung der Beckeneingangsebene bei dieser speziellen Lage des Patienten aus.

Es ist Aufgabe der Erfindung, ein Verfahren anzugeben, wie auch in diesen Fällen, in denen einer der Punkte A oder B nicht zugänglich ist, die Beckeneingangsebene bestimmt werden kann.

Diese Aufgabe wird bei einem Verfahren der eingangs beschriebenen Art erfindungsgemäß dadurch gelöst, dass man zusätzlich nichtinvasiv die Lage der folgenden Punkte des Beckenknochens bestimmt:
- Punkt D :: spina iliaca posterior superior links (linker hinterer oberer Darmbeinstachel)
- Punkt E :: spina iliaca posterior superior rechts (rechter hinterer oberer Darmbeinstachel)
und dass man aus der Lage der nichtinvasiv bestimmten Punkte A oder B, C, D und E die Lage der Beckeneingangsebene berechnet.

Die nicht mögliche Bestimmung des fehlenden Punktes A oder B wird also ersetzt durch die auch in der Seitenlage des Patienten mögliche Bestimmung zusätzlicher Punkte, nämlich der Punkte D und E, und aus diesen Punkten können durch bestimmte Rechenverfahren die Lagedaten der Beckeneingangsebene errechnet werden. Dabei ist davon auszugehen, dass zwischen der Lage der Punkte D und E einerseits und der Lage der Punkte A und B andererseits am Beckenknochen geometrische Beziehungen bestehen, die bei den Beckenknochen aller Patienten sehr ähnlich oder gleich sind.

Die nichtinvasive Bestimmung der Lagedaten der beschriebenen Punkte lässt sich beispielsweise durch perkutanes Tasten erreichen, dieses Tasten kann auch mit Hilfe eines navigierten Tastinstrumentes erfolgen. Eine andere Möglichkeit liegt darin, die Lage mittels eines navigierten Ultraschallsensors zu bestimmen. Auch andere, dem Fachmann geläufige Methoden zur nichtinvasiven Bestimmung von knöchernen Strukturen können Anwendung finden, beispielsweise bildgebende Verfahren mit Röntgenstrahlen oder durch Kernspin-Resonanz.

Bei einer ersten bevorzugten Ausführungsform der Erfindung wird so vorgegangen, dass man zur Berechnung der Lage der Beckeneingangsebene den Vektor zwischen den Punkten D und E so parallel zu sich selbst verschiebt, dass er durch den nichtinvasiv bestimmten Punkt A oder B hindurch geht, und dass man die Beckeneingangsebene bestimmt als die Ebene, die durch den Vektor zwischen dem nichtinvasiv bestimmten Punkt A oder B und dem Punkt C und den von dem nichtinvasiv bestimmten Punkt A oder B ausgehenden, zu dem Vektor zwischen den Punkten D und E parallelen Vektor aufgespannt wird.

Man geht also davon aus, dass der Verbindungsvektor zwischen den Punkten D und E einerseits und den Punkten A und B andererseits bei allen Patienten im wesentlichen parallel verlaufen, so dass man durch eine Verschiebung des Vektors, der die Punkte D und E verbindet, in den nichtinvasiv bestimmten Punkt A oder B diesen Vektor in die Beckeneingangsebene verschiebt. Damit können durch diesen Vektor und den Verbindungsvektor zwischen dem nichtinvasiv bestimmten Punkt A oder B und dem Punkt C die Lagedaten der Beckeneingangsebene bestimmt werden.

Bei diesem Verfahren ist es nicht notwendig, die exakte Lage des unzugänglichen und nicht bestimmbaren Punktes B oder A zu kennen, da allein durch die Anordnung der beiden Vektoren die Beckeneingangsebene ausreichend bestimmt ist.

Dabei ist es auch unerheblich, ob der parallel zu dem Vektor zwischen den Punkten D und E verschobene Vektor von dem nichtinvasiv bestimmten Punkt A oder B ausgeht oder lediglich durch diesen hindurch geht, wesentlich ist nur, dass dieser nichtinvasiv bestimmte Punkt A oder B auf diesem verschobenen Vektor liegt.

Bei einem abgewandelten Verfahren wird so vorgegangen, dass man zur Berechnung der Lage des unbekannten Punktes B oder A den Vektor zwischen den Punkten D und E so verschiebt, dass er von dem nichtinvasiv bestimmten Punkt A oder B ausgeht und auf den unbekannten Punkt B oder A weist, die Länge des Vektors mit einem diesen verlängernden Faktor multipliziert und als Lage des unbekannten Punktes B oder A die Spitze des derart verlängerten Vektors annimmt. Bei diesem Verfahren geht man davon aus, dass einmal die Richtung des Vektors zwischen den Punkten D und E und des Vektors zwischen den Punkten A und B parallel angeordnet ist, und zum anderen davon, dass es bestimmte Längenverhältnisse zwischen diesen Vektoren gibt, die für eine große Anzahl von Patienten einen sehr ähnlichen oder sogar den gleichen Wert aufweisen, so dass aus dem Abstand zwischen den Punkten D und E Rückschlüsse auf den Abstand zwischen den Punkten A und B möglich sind.

Dieser Faktor kann einen festen Wert aufweisen, der beispielsweise zwischen 2,8 und 2,5 liegt. Dabei kann dieser Wert für Männer und Frauen unterschiedlich sein, beispielsweise kann der Wert bei Männern zwischen 2,8 und 2,75 liegen, bei Frauen zwischen 2,63 und 2,58.

Bei einem anderen Verfahren wird dieser Faktor dadurch bestimmt, dass der Abstandsvektor des nichtinvasiv bestimmten Punktes A oder B und des Punktes C auf den verschobenen Vektor projiziert und dass die projizierte Strecke längs des verschobenen Vektors verdoppelt wird. Bei diesem Verfahren wird davon ausgegangen, dass das Becken zu beiden Seiten der symphysis pubis symmetrisch aufgebaut ist, so dass sich die Länge des Verbindungsvektors zwischen den Punkten A und B als doppelte Strecke zwischen dem nichtinvasiv bestimmten Punkt A oder B einerseits und der Projektion der symphysis pubis auf diesen Vektor andererseits ergibt.

Bei beiden Verfahren ist es möglich, ohne Kenntnis eines der Punkte A oder B dessen Lage relativ genau zu bestimmen, so dass die Beckeneingangsebene aus den drei Punkten A, B und C auch ohne die Möglichkeit bestimmbar ist, die Lagedaten eines der Punkte A und B direkt zu erfassen.

Der Erfindung liegt weiterhin die Aufgabe zugrunde, eine Vorrichtung zur Durchführung dieses Verfahrens anzugeben.

Diese Aufgabe wird erfindungsgemäß gelöst durch eine Vorrichtung zur Bestimmung der Frontalebene des Beckenknochens, die definiert wird durch folgende drei Punkte des Beckenknochens:
- Punkt A :: spina iliaca anterior superior links (linker vorderer oberer Darmbeinstachel)
- Punkt B :: spina iliaca anterior superior rechts (rechter vorderer oberer Darmbeinstachel)
- Punkt C :: symphysis pubis (Schambein)
und mit einem Navigationssystem, einem navigierten Lagesensor für die Punkte A, B und C und für folgende weitere Punkte
- Punkt D :: spina iliaca posterior superior links (linker hinterer oberer Darmbeinstachel)
- Punkt E :: spina iliaca posterior superior rechts (rechter hinterer oberer Darmbeinstachel),
und mit einer Datenverarbeitungsanlage, die so programmiert ist, dass sie aus den Lagedaten der Punkte A oder B, C, D und E die Lage der Beckeneingangsebene berechnet.

Der Lagesensor kann ein Tastinstrument sein, insbesondere ein navigiertes Tastinstrument, ein Ultraschallsensor, insbesondere ein navigierter Ultraschall-sensor oder ein anderes Instrument, welches ermöglicht, nichtinvasiv oder gegebenenfalls auch invasiv die Lage der Punkte A, B, C, D und E zu erfassen, sofern sie im Hinblick auf die Lage des Patienten zugänglich sind.

Die Datenverarbeitungsanlage kann dabei so programmiert sein, dass sie zur Berechnung der Lage der Beckeneingangsebene den Vektor zwischen den Punkten D und E so parallel zu sich selbst verschiebt, dass er durch den nichtinvasiv bestimmten Punkt A oder B hindurchgeht, und dass sie die Beckeneingangsebene bestimmt als die Ebene, die durch den Vektor zwischen dem nichtinvasiv bestimmten Punkt A oder B und dem Punkt C und den von dem nichtinvasiv bestimmten Punkt A oder B ausgehenden, zu dem Vektor zwischen den Punkten D und E parallelen Vektor aufgespannt wird.

Bei einer anderen Ausgestaltung der Erfindung kann die Datenverarbeitungsanlage so programmiert sein, dass sie zur Berechnung der Lage des unbekannten Punktes B oder A den Vektor zwischen den Punkten D und E so verschiebt, dass er von dem nichtinvasiv bestimmten Punkt A oder B ausgeht und in Richtung auf den unbekannten Punkt B oder A weist, die Länge des Vektors mit einem diesen verlängernden Faktor multipliziert und als Lage des unbekannten Punktes B oder A die Spitze des derart verlängerten Vektors annimmt.

In beiden Fällen wird davon ausgegangen, dass der die Punkte A und B verbindende Vektor und der die Punkte D und E verbindende Vektor bei unterschiedlichen Patienten annähernd oder exakt parallel verlaufen. Bei der ersten Programmierungsart wird die Beckeneingangsebene bestimmt, ohne dass man die Lage des nicht bestimmbaren Punktes B oder A ermittelt, bei dem anschließend beschriebenen Verfahren jedoch kann auch die Lage dieses zweiten, nicht unmittelbar bestimmbaren Punktes B oder A berechnet werden.

Bei dieser Methode verwendet die Datenverarbeitungsanlage einen Faktor, um den Vektor zu verlängern, der von dem nichtinvasiv bestimmten Punkt A oder B ausgeht und der parallel zu den Punkten D und E verläuft. Dieser Faktor kann dabei einen festen Wert aufweisen, der insbesondere zwischen 2,8 und 2,5 liegt.

Dieser Wert kann bei Männern und Frauen verschieden sein, beispielsweise kann er bei Männern zwischen 2,8 und 2,75 liegen, bei Frauen zwischen 2,63 und 2,58.

Die Datenverarbeitungsanlage kann aber auch so programmiert sein, dass der Faktor dadurch bestimmt wird, dass der Abstandsvektor des nichtinvasiv bestimmten Punktes A oder B und des Punktes C auf den verschobenen Vektor projiziert wird und dass die projizierte Strecke längs des verschobenen Vektors verdoppelt wird. In diesem Falle wird davon ausgegangen, dass der Beckenknochen zu der von der symphysis pubis definierten Mittellage symmetrisch aufgebaut ist.

Die nachfolgende Beschreibung bevorzugter Ausführungsformen der Erfindung dient im Zusammenhang mit der Zeichnung der näheren Erläuterung. Es zeigen:
- Figur 1 :: eine schematische Darstellung eines in Seitenlage gelagerten Patienten und eines Navigationssystems zur Lagebestimmung navigierter Lagesensoren;
- Figur 2 :: eine schematische Frontalansicht eines Patienten mit den die Beckeneingangsebene definierenden Punkten A, B und C und
- Figur 3 :: eine schematische Schnittansicht des Beckenknochens in einer Ansicht von oben mit den für die Bestimmung der Beckeneingangsebene relevanten Punkten A, B, C, D und E.

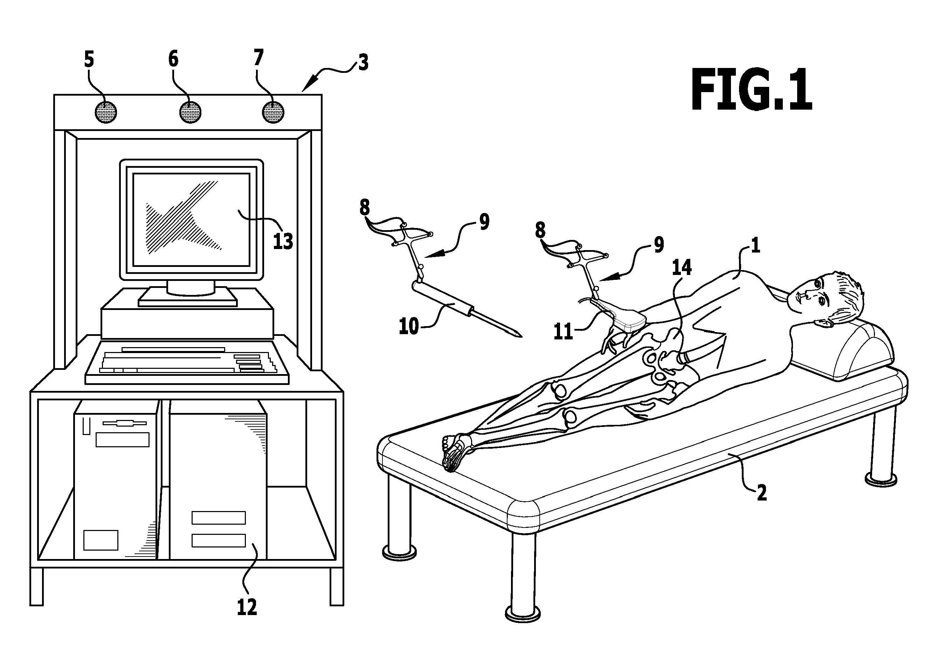

In Figur 1 ist ein Patient 1 auf einem Operationstisch 2 in Seitenlage dargestellt. In dieser Lage können Operationen an dem Patienten ausgeführt werden. Neben dem Operationstisch 2 ist ein Navigationssystem 3 angeordnet mit mehreren Strahlungssendern 5, 6, 7, die auch gleichzeitig als Strahlungsempfänger ausgebildet sind. Bei der ausgesendeten Strahlung kann es sich um Infrarotstrahlung handeln.

Diese Strahlung wird von Reflexionsflächen 8 reflektiert, dabei kann es sich um Kugeln handeln, die als Markierelemente 9 an diversen Instrumenten 10 angeordnet sein können, beispielsweise einem Tastinstrument 10 und einem Ultraschallsensor 11. Das Navigationssystem kann in an sich bekannter Weise dadurch die Lage der Instrumente im Raum bestimmen, also ihre exakte Position und ihre Orientierung.

Dem Navigationssystem 3 ist außerdem eine Datenverarbeitungsanlage 12 zugeordnet mit einer Anzeigeeinrichtung 13, im dargestellten Ausführungsbeispiel in Form eines Monitors.

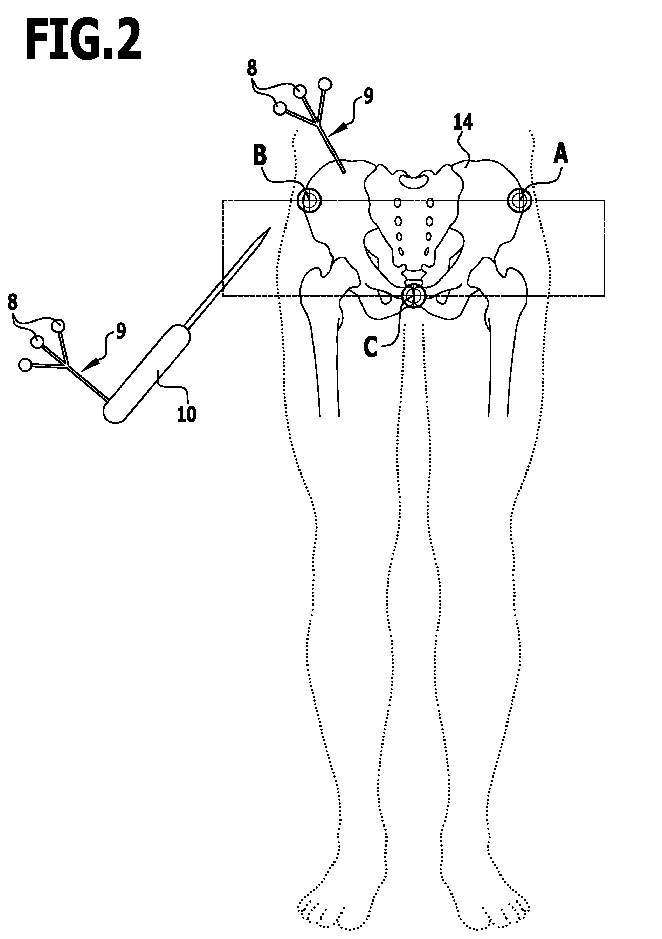

Um die Lage des Beckenknochens 14 eines Patienten beschreiben zu können, werden drei markante Punkte des Beckenknochens verwendet, die eine so genannte Beckeneingangsebene 15 definieren (Figur 2). Bei diesen markanten Punkten handelt es sich um die folgenden Punkte:
- Punkt A :: spina iliaca anterior superior links (linker vorderer oberer Darmbeinstachel)
- Punkt B :: spina iliaca anterior superior rechts (rechter vorderer oberer Darmbeinstachel)
- Punkt C :: symphysis pubis (Schambein).

Diese drei Punkte lassen sich perkutan tasten, beispielsweise manuell oder mit Hilfe des navigierten Tastinstrumentes 10. Es ist auch möglich, diese Punkte mit Hilfe des navigierten Ultraschallsensors zu bestimmen, in jedem Fall kann auf diese Weise die Lage der drei Punkte A, B und C im Raum festgestellt werden, und das Navigationssystem kann einen Datensatz, der der Lage der Punkte A, B und C entspricht, an die Datenverarbeitungsanlage 12 abgeben. Allerdings setzt dies voraus, dass alle drei Punkte A, B und C frei zugänglich sind. Dies ist in der in Figur 1 dargestellten Seitenlage jedoch nicht der Fall, so dass in diesem Falle nur jeweils zwei der drei Punkte in der beschriebenen Weise bestimmt werden können, nämlich entweder der Punkt A oder der Punkt B und zusätzlich in jedem Falle der Punkt C.

Damit ist es bei Patienten in Seitenlage zunächst nicht möglich, die Beckeneingangsebene zu bestimmen.

Aus diesem Grunde bestimmt der Operateur zusätzlich zu den beiden bestimmbaren Punkten A oder B und C noch zwei weitere markante Punkte des Beckenknochens 14, nämlich die folgenden Punkte:
- Punkt D :: spina iliaca posterior superior links (linker hinterer oberer Darmbeinstachel)
- Punkt E :: spina iliaca posterior superior rechts (rechter hinterer oberer Darmbeinstachel).

Diese beiden markanten Punkte sind bei einem in Seitenlage liegenden Patienten an dessen Rücken ohne weiteres durch Tasten oder mit Hilfe einer Ultraschallsonde etc. lokalisierbar.

Für die Berechnung der Beckeneingangsebene stehen also nach einer solchen Messung folgende Punkte zur Verfügung: A, C, D, E oder B, C, D und E. Dabei liegen die Punkte D und E nicht in der Beckeneingangsebene, sie können jedoch entsprechend dem nachstehend beschriebenen Verfahren verwendet werden, um die Lage der Beckeneingangsebene oder aber auch den fehlenden Punkt B beziehungsweise A in der Beckeneingangsebene hinsichtlich ihrer Lage zu bestimmen.

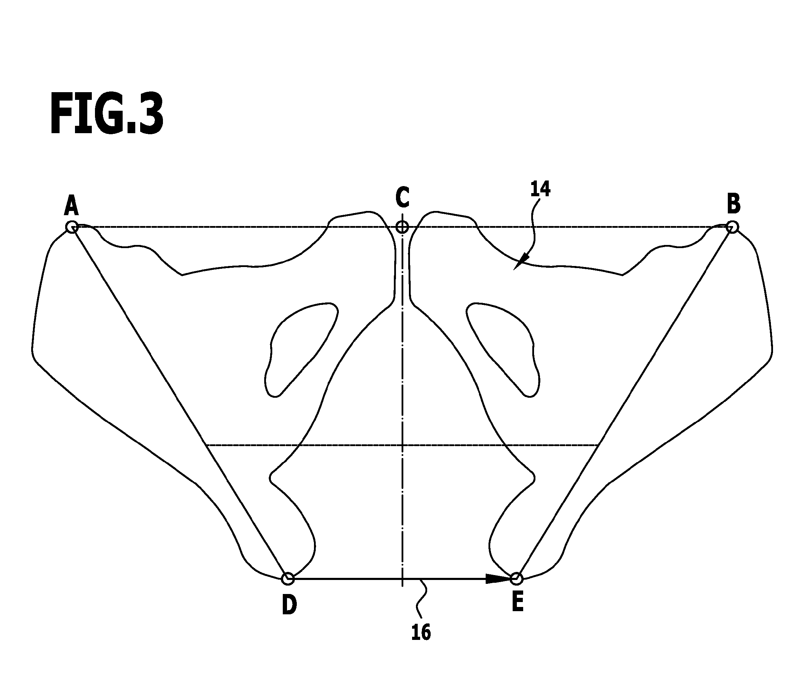

Dazu wird zunächst von der Datenverarbeitungsanlage der Vektor bestimmt, der die Punkte D und E verbindet. Dieser Vektor ist in Figur 3 mit dem Bezugszeichen 16 gekennzeichnet. Dieser Vektor 16 wird parallel verschoben, und zwar bei einem ersten Verfahren derart, dass dieser parallel verschobene Vektor durch den nichtinvasiv bestimmten Punkt A oder B hindurchgeht.

Dieser auf diese Weise bestimmte Vektor und der den nicht invasiv bestimmten Punkt A oder B und den Punkt C verbindende Vektor spannen gemeinsam eine Ebene auf, und diese Ebene ist die Beckeneingangsebene, die gesucht ist. Damit kann die Datenverarbeitungsanlage aus diesen beiden Vektoren die Lage der Beckeneingangsebene berechnen. Als Ergebnis erhält man die Lage der Beckeneingangsebene, nicht aber die genauen Koordinaten des unzugänglichen und hinsichtlich seiner Lagedaten nicht bestimmbaren Punktes B oder A.

Diese Daten können bei einem anderen Verfahren in der folgenden Weise erhalten werden:

Wie bei dem oben beschriebenen Verfahren wird zunächst von der Datenverarbeitungsanlage der Vektor bestimmt, der die Punkte D und E verbindet, also der Vektor 16 in Figur 3.

Dieser Vektor 16 wird parallel verschoben, so dass sein Anfangspunkt in dem Punkt A oder B liegt, dessen Lagedaten bestimmt werden konnten. Außerdem wird der Vektor 16 gegebenenfalls so um 180° gedreht, dass seine Spitze in Richtung des jeweils anderen Punktes B beziehungsweise A gerichtet ist, dessen Lagedaten nicht bestimmt werden konnten.

Da der Abstand zwischen den Punkten D und E deutlich kleiner ist als der Abstand zwischen den Punkten A und B, weist damit zwar der Vektor 16 in Richtung des Punktes B beziehungsweise A, dessen Lagedaten bisher nicht bestimmt werden konnten, die Spitze hat aber einen Abstand von diesem Punkt. Es ist also noch notwendig, die Länge dieses verschobenen Vektors zu modifizieren.

Dies kann in verschiedener Weise erfolgen.

Bei einem ersten Verfahren wird angenommen, dass zwischen dem Abstand der Punkte D und E einerseits und der Punkte A und B andererseits ein fester Faktor existiert, der für alle Beckenknochen verschiedener Patienten gleich oder ähnlich ist. Es hat sich herausgestellt, dass dies annähernd der Fall ist, wobei dieser Faktor für Männer und Frauen verschieden ist. Bei Männern liegt dieser Faktor etwa zwischen 2,8 und 2,75, bei Frauen etwa zwischen 2,63 und 2,58. Verwendet man einen solchen Faktor, um aus der Länge des Vektors 16 den Abstand zwischen den Punkten A und B zu bestimmen, so erhält man in der Praxis Werte, bei denen die Standardabweichung gegenüber den tatsächlichen Werten des Abstandes zwischen den Punkten A und B in der Größenordnung zwischen 10 und 15 mm liegt, man erhält also eine relativ gute Annäherung der Lage des nicht direkt bestimmbaren dritten Punktes der Beckeneingangsebene.

Bei einem zweiten Verfahren wird davon ausgegangen, dass der Beckenknochen 14 bei jedem Patienten zu einer senkrechten Mittelebene symmetrisch ist, die durch die symphysis pubis hindurch geht. Es wird daher der Vektor, der den gemessenen der beiden Punkte A und B mit dem Punkt C verbindet, auf den Vektor projiziert, der sich aus dem verschobenen Vektor 16 ergibt und der bei dem gemessenen Punkt A oder B beginnt. Damit wird wegen der angenommenen Symmetrie die Spitze des vom gemessenen Punkt A oder B zum Punkt C verlaufenden Vektors in einem Punkt auf den verschobenen Vektor projiziert, der dem halben Abstand zwischen den Punkten A und B entspricht. Es genügt nun, diesen Abstand zu verdoppeln, um zu dem nicht bestimmbaren Punkt B oder A zu gelangen, so dass damit die Lagedaten auch dieses Punktes zur Verfügung stehen.

## Patentansprüche

1. Verfahren zur Bestimmung der Beckeneingangsebene des Beckenknochens, die definiert wird durch folgende drei Punkte des Beckenknochens:
Punkt A : spina iliaca anterior superior links (linker vorderer oberer Darmbeinstachel)
Punkt B : spina iliaca anterior superior rechts (rechter vorderer oberer Darmbeinstachel)
Punkt C : symphysis pubis (Schambein)
durch nichtinvasive Bestimmung der Lage einer der beiden Punkte A oder B und des Punktes C, **dadurch gekennzeichnet, dass** man zusätzlich nichtinvasiv die Lage der folgenden Punkte des Beckenknochens bestimmt:
Punkt D : spina iliaca posterior superior links (linker hinterer oberer Darmbeinstachel)
Punkt E : spina iliaca posterior superior rechts (rechter hinterer oberer Darmbeinstachel)
und dass man aus der Lage der nichtinvasiv bestimmten Punkte A oder B, C, D, E die Lage der Beckeneingangsebene berechnet.

2. Verfahren nach Anspruch 1, **dadurch gekennzeichnet, dass** man die Lage der Punkte A oder B, C, D, E durch perkutanes Tasten bestimmt.

3. Verfahren nach Anspruch 1, **dadurch gekennzeichnet, dass** man die Lage der Punkte A oder B, C, D, E durch eine Ultraschall-Untersuchung bestimmt.

4. Verfahren nach einem der voranstehenden Ansprüche, **dadurch gekennzeichnet, dass** man zur Berechnung der Lage der Beckeneingangsebene den Vektor zwischen den Punkten D und E so parallel zu sich selbst verschiebt, dass er durch den nichtinvasiv bestimmten Punkt A oder B hindurchgeht, und dass man die Beckeneingangsebene bestimmt als die Ebene, die durch den Vektor zwischen dem nichtinvasiv bestimmten Punkt A oder B und dem Punkt C und den von dem nichtinvasiv bestimmten Punkt A oder B ausgehenden, zu dem Vektor zwischen den Punkten D und E parallelen Vektor aufgespannt wird.

5. Verfahren nach einem der Ansprüche1 bis 3, **dadurch gekennzeichnet, dass** man zur Berechnung der Lage des unbekannten Punktes B oder A den Vektor zwischen den Punkten D und E so parallel zu sich selbst verschiebt, dass er von dem nichtinvasiv bestimmten Punkt A oder B ausgehend auf den unbekannten Punkt B oder A weist, die Länge des verschobenen Vektors mit einem diesen verlängernden Faktor multipliziert und als Lage des unbekannten Punktes B oder A die Spitze des derart verlängerten Vektors annimmt.

6. Verfahren nach Anspruch 5, **dadurch gekennzeichnet, dass** der Faktor einen festen Wert aufweist.

7. Verfahren nach Anspruch 6, **dadurch gekennzeichnet, dass** der Faktor zwischen 2,8 und 2,5 liegt.

8. Verfahren nach Anspruch 7, **dadurch gekennzeichnet, dass** der Faktor bei Männern zwischen 2,8 und 2,75 liegt.

9. Verfahren nach Anspruch 7, **dadurch gekennzeichnet, dass** der Faktor bei Frauen zwischen 2,63 und 2,58 liegt.

10. Verfahren nach Anspruch 5, **dadurch gekennzeichnet, dass** der Faktor **dadurch** bestimmt wird, dass der Abstandsvektor des nichtinvasiv bestimmten Punktes A oder B und des Punktes C auf den verschobenen Vektor projiziert wird und dass die projizierte Strecke längs des verschobenen Vektors verdoppelt wird.

11. Vorrichtung zur Bestimmung der Beckeneingangsebene (15) des Beckenknochens (14), die definiert wird durch folgende drei Punkte des Beckenknochens (14):
Punkt A : spina iliaca anterior superior links (linker vorderer oberer Darmbeinstachel)
Punkt B : spina iliaca anterior superior rechts (rechter vorderer, oberer Darmbeinstachel)
Punkt C : symphysis pubis (Schambein)
mit einem Navigationssystem (3), einem navigierten Lagesensor für die Punkte A, B und C und für folgende weitere Punkte:
Punkt D : spina iliaca posterior superior links (linker hinterer, oberer Darmbeinstachel)
Punkt E : spina iliaca posterior superior rechts (rechter hinterer, oberer Darmbeinstachel)
und mit einer Datenverarbeitungsanlage (12), die so programmiert ist, dass sie aus den Lagedaten der Punkte A oder B, C, D und E die Lage der Beckeneingangsebene (15) berechnet.

12. Vorrichtung nach Anspruch 11, **dadurch gekennzeichnet, dass** der Lagesensor ein Tastinstrument (10) ist.

13. Vorrichtung nach Anspruch 11, **dadurch gekennzeichnet, dass** der Lagesensor ein Ultraschallsensor (11) ist.

14. Vorrichtung nach einem der Ansprüche 11 bis 13, **dadurch gekennzeichnet, dass** die Datenverarbeitungsanlage (12) so programmiert ist, dass sie zur Berechnung der Lage der Beckeneingangsebene den Vektor zwischen den Punkten D und E so parallel zu sich selbst verschiebt, dass er durch den nichtinvasiv bestimmten Punkt A oder B hindurchgeht, und dass sie die Beckeneingangsebene berechnet als die Ebene, die durch den Vektor zwischen dem nichtinvasiv bestimmten Punkt A oder B und dem Punkt C und den von dem nichtinvasiv bestimmten Punkt A oder B ausgehenden, zu dem Vektor zwischen den Punkten D und E parallelen Vektor aufgespannt wird.

15. Vorrichtung nach einem der Ansprüche 11 bis 13, **dadurch gekennzeichnet, dass** die Datenverarbeitungsanlage (12) so programmiert ist, dass sie zur Berechnung der Lage des unbekannten Punktes B oder A den Vektor (16) zwischen den Punkten D und E so parallel zu sich selbst verschiebt, dass er von dem bestimmbaren Punkt A oder B ausgehend auf den unbekannten Punkt B oder A weist, die Länge des Vektors (16) mit einem diesen verlängernden Faktor multipliziert und als Lage des unbekannten Punktes B oder A die Spitze des derart verlängerten Vektors annimmt.

16. Vorrichtung nach Anspruch 15, **dadurch gekennzeichnet, dass** der Faktor einen festen Wert aufweist.

17. Vorrichtung nach Anspruch 16, **dadurch gekennzeichnet, dass** der Faktor zwischen 2,8 und 2,5 liegt.

18. Vorrichtung nach Anspruch 17, **dadurch gekennzeichnet, dass** der Faktor bei Männern bei etwa zwischen 2,8 und 2,75 liegt.

19. Vorrichtung nach Anspruch 17, **dadurch gekennzeichnet, dass** der Faktor bei Frauen zwischen 2,63 und 2,58 liegt.

20. Vorrichtung nach Anspruch 15, **dadurch gekennzeichnet, dass** der Faktor **dadurch** bestimmt wird, dass der Abstandsvektor des bestimmbaren Punktes A oder B und des Punktes C auf den verschobenen Vektor projiziert wird und dass die projizierte Strecke längs des verschobenen Vektors verdoppelt wird.
